# EUROPEAN PATENT SPECIFICATION

(11) **EP 2 943 525 B1**
(45) Date of publication and mention of the grant of the patent: **05.10.2016**
(21) Application number: 14714598.1
(22) Date of filing: 13.01.2014
(51) Int. Cl.: C08G 83/00, B01J 23/02, C08G 65/34

(54) **METHOD FOR THE PRODUCTION OF HYPERBRANCHED POLYGLYCEROL**
VERFAHREN ZUR HERSTELLUNG VON HOCHVERZWEIGTEM POLYGLYCEROL
PROCÉDÉ DE PRODUCTION D'UN POLYGLYCÉROL HYPERRAMIFIÉ

(30) Priority: 13.01.2013 EP 13151096
(43) Date of publication of application: 18.11.2015
(73) Proprietor: de Schrijver, Aster, 9831 Deurle (BE)
(72) Inventor: CIRIMINNA, Rosaria, I-90146 Palermo (IT); PAGLIARO, Mario, I-90146 Palermo (IT)
(74) Representative: BiiP cvba
(86) International application number: PCT/EP2014/050438
(87) International publication number: WO 2014/108524

(56) References cited:
- EP-A1- 1 961 726
- WO-A2-2010/044531
- AGNIESZKA&EMSP14;M. RUPPERT ET AL: "Glycerol Etherification over Highly Active CaO-Based Materials: New Mechanistic Aspects and Related Colloidal Particle Formation", CHEMISTRY - A EUROPEAN JOURNAL, vol. 14, no. 7, 27 February 2008 (2008-02-27), pages 2016-2024, XP055054897, ISSN: 0947-6539, DOI: 10.1002/chem.200701757
- ANDREW GOODWIN ET AL: "Inimer Mediated Synthesis of Hyperbranched Polyglycerol via Self-Condensing Ring-Opening Polymerization", MACROMOLECULES, vol. 45, no. 24, 21 December 2012 (2012-12-21), pages 9657-9665, XP055054933, ISSN: 0024-9297, DOI: 10.1021/ma301768k

## Description

### TECHNICAL FIELD

The present invention concerns a method for producing hyperbranched polyglycerol by polycondensation of glycerol in presence of a specific catalyst.

### BACKGROUND OF THE INVENTION

Hyperbranched polyglycerol (hbPG) is a highly branched polyol possessing an inert polyether scaffold whose high functionality, in combination with the versatile and well-investigated reactivity of its hydroxyl functionality, forms the basis of a variety of useful derivatives (cf. Frey, H.; and Haag, R. "Dendritic Polyglycerol: A New Versatile Biocompatible Material," Rev. Mol. Biotech, 90 (2002) 257-267). Each branch ends in a hydroxyl function, which renders hyperbranched polyglycerol a highly functional material; for example, a molecule with a molecular weight of 5,000 g mol⁻¹ may possess 68 hydroxyl end-groups. A number of polyglycerols are commercially available, with applications ranging from cosmetics to controlled drug release. For example, partial esterification of polyglycerol with fatty acids yields amphiphilic materials which behave as nanocapsules. Such nanocapsules can for example incorporate polar molecules as guests and solubilize them in a nonpolar environment. Biocompatibility is an attractive feature of aliphatic polyether structures containing hydroxyl end-groups, including polyglycerols or linear polyethylene glycols (PEGs), which are approved for a wide variety of medical and biomedical applications.

Today, hyperbranched polyglycerol is mostly produced industrially by controlled etherification of glycerol via anionic, ring-opening multibranching polymerization of glycidol under slow monomer addition. These conditions yield polymers with low and narrow polydispersities (M_{w}/Mₙ = 1.2-1.9) and with a number average molecular weight (Mₙ) of up to 24,000 g mol⁻¹. In detail, hyperbranched polyglycerols are typically prepared by ring opening polymerization of glycidol either by cationic or anionic means (cf. R. Tokar, P. Kubisa and S. Penczek, "Cationic polymerization of glycidol: coexistence of the activated monomer and active chain end mechanism", Macromolecules, 27 (1994) 320-322) and A. Sunder, R. Hanselmann, H. Frey, R. Mülhaupt, "Controlled synthesis of hyperbranched polyglycerols by ring-opening multibranching polymerization", Macromolecules, 32 (1999) 4240- 4246).

The number and scope of the applications of this versatile material is huge and gowing (cf. Wilms, D.; Stiriba, S.-E.; and Frey, H. "Hyperbranched Polyglycerols: From the Controlled Synthesis of Biocompatible Polyether Polyols to Multipurpose Applications" Acc. Chem. Res. 43 (2010), 129-141). Recently, for example, the properties of dendritic polyglycerol sulfates as potent inhibitors of inflammation have been reported (cf. Haag, R. et al. "Dendritic polyglycerol sulfates as multivalent inhibitors of inflammation" Proc. Natl. Acad. Sci. USA 107 (2010) 19679-19684).

German patent application DE10211664A1 discloses a method for manufacturing such polyglycerol polymers, comprising a step of polymerizing the glycidol in a solution in an aqueous medium in the presence of a water soluble basic catalyst and a chemical change step for making the polyglycerol polymers water soluble. WO2009153529 discloses an alternative method for manufacturing acrolein polymers, comprising a first step for dehydrating glycerol from a glycerol aqueous solution in the presence of an acid catalyst, followed by a polymerization of the obtained substance in the presence of an ionic catalyst or a free radical initiator.

One drawback of the foregoing methods is that traditional synthetic techniques start from expensive glycidol, a cyclic compound that requires epoxidation of allyl alcohol. Moreover, glycidol is listed as an IARC group 2A carcinogen, and is a toxic compound irritant of the skin, eyes, mucous membranes, and upper respiratory tract. Clearly a route to hyperbranched polyglycerol process starting from non toxic and readily available glycerol would be highly desirable. Besides being non-toxic, glycerol is currently obtained in huge amounts (2 million t/yr) as by-product of biodiesel manufacturing, as well as from the production of soaps and free fatty acids (cf. M. Pagliaro and M. Rossi, "The Future of Glycerol;" RSC Publishing, Cambridge: 2010).

All the methods for producing hyperbranched polyglycerol reviewed *supra* start from the ring-opening multibranching polymerization (ROMBP) of glycidol or involve glycidol or glycidol derivatives such as glycidyl methacrylate (cf. M. Hu et al., "Biodegradable Hyperbranched Polyglycerol with Ester Linkages for Drug Delivery", Biomacromolecules 11 (2012), 3552-3561).

Another drawback of the ROMBP synthetic approach is that it only affords polymers with a maximum size of approximately 3-10 nm, whereas the optimum size for nanoparticle biomedical applications is a diameter in the range of 25-100 nm. Recently, a preparation of polyglycerol particles on different length scales was proposed by extending - through miniemulsion templating spanning structures ranging from hyperbranched polyglycerols (3 nm) to nanogels (32 nm) and microgels (140 and 220 mm) (cf. Dirk Steinhilber, Sebastian Seiffert, John A. Heyman, Florian Paulus, David A. Weitz Rainer Haag, "Hyperbranched polyglycerols on the nanometer and micrometer scale," Biomaterials, 32 (2011) 1311-1316).

Linear polyglycerols are usually obtained by alkaline binary metal oxide catalysis of glycerol etherification. WO2010/044531, for example, describes a CaO based etherification process capable to produce high yields of linear polyglycerol. Linear polyglycerols are suitable and approved as cosmetic or food additives. The special properties of a similar nanostructured CaO in glycerol etherification to diglycerol at 220 °C in the absence of a solvent under Ar were recently reported (cf. A. M. Ruppert, J. D. Meeldijk, B. W. M. Kuipers, B. H. Erné, B. M. Weckhuysen, "Glycerol etherification over highly active CaO-based materials: new mechanistic aspects and related colloidal particle formation", Chem. Eur. J. 14 (2008) 2016-2024), whereas commercial CaO is poorly active. No route based on conventional or nanostructured CaO catalysts to hyperbranched polyglycerol has, to the best of our knowledge, been yet reported. The present invention provides a method for producing dendritic polyglycerol which is cheap, simple, green, and having a high yield. This and other advantages of the present invention are presented in continuation.

### SUMMARY OF THE INVENTION

The present invention is defined in the appended independent claims. Preferred embodiments are defined in the dependent claims. In particular, the present invention concerns a process for producing hyperbranched polyglycerol from glycerol comprising the following steps:
(a) Adding in a vessel glycerol and a CaO-based catalyst,
(b) Flushing an inert gas, preferably carbon dioxide, to the resulting mixture and hermetically closing the reaction vessel, allowing pressure to build up from 1 to 10 bar above atmospheric, preferably from 2 to 6 bar;
(c) Heating the reaction mixture at a reaction temperature of at least 100°C and below its boiling point;
(d) Maintaining the reaction conditions until at least 40 wt.% of glycerol is polycondensed and converted into hyperbranched polyglycerol, with concomitant water formation, ,
(e) Separating the dendritic polyglycerol from other components present in the mixture; such as water, catalyst and reaction by-products such as hydroxyacids and glycerol carbonate esters typically obtained e.g., by condensation of deprotonated glycerol, and carbon dioxide.
(f) **Characterized in that,** the calcium based catalyst is nanostructured calcium oxide in the form of a powder of mean particle size typically smaller than 100 nm measured according to ASTM D4464. In the present context, the expressions "hyperbranched polyglycerol" and "dendritic polyglycerol" are used as synonyms and refer to polyglycerols characterized by the combination of a stable, biocompatible polyether scaffold, high-end group functionality and a compact, well-defined dendrimer-like architecture, i.e., in the form of a repetitively branched molecule.

It is preferred that the calcium based catalyst has a strong Lewis acidity as it denotes a high activity. The Lewis activity of the catalyst can be characterized by measuring the blue shift, Δv8a, of the C=C stretching band of adsorbed pyridine as compared to the value of 1580 cm⁻¹ for gas-phase pyridine. The blue shift of the Ca oxide catalyst of the present invention is preferably comprised between 14 and 20 cm⁻¹, more preferably, between 16 and 19 cm⁻¹, most preferably between 1.5 and 18.5 cm⁻¹. The BET specific surface area of the nanostructured CaO catalyst of the present invention is preferably greater than 50 m² / g, more preferably comprised between 60 and 100 m² / g. The nanostructured catalyst is in the form of a powder of mean particle size generally smaller than 100 nm, preferably less than 50 nm, more preferably less than 20 nm.

In one embodiment, the nanostructured Ca oxide is obtained by heating Ca(NO₃)₂ 4H₂O at a temperature of at least 500°C for at least 50 min. In an alternative embodiment, Ca(OH)₂ can be heated at a temperature of at least 350°C for a period of up to 12 h. In yet an alternative embodiment, the nanostructured Ca oxide is obtained by:
- Hydrolysing Ca(OCH₃)₂ in solution in an organic solvent by addition of water;
- Forming Ca(OH)₂ by treating the hydrolysed solution in an autoclave at a temperature of at least 200°C under elevated pressure;
- Converting the thus obtained Ca(OH)₂ into CaO by a heat treatment at least 300°C under reduced pressure below 10⁻² Torr.

The great advantage of the present invention is that conversion of glycerol into hyperbranched polyglycerol can be performed in the liquid phase, substantially free of solvents or of an aqueous medium. In a preferred embodiment, the calcium based catalyst is present in the mixture in an amount comprised between 4 and 30 mol.% with respect of the total weight of glycerol and catalyst, preferably between 5 and 25 mol.%, more preferably between 10 and 20 mol.%. The reaction temperature at step (c) is preferably at least 120°C, more preferably at least 160°C, most preferably at least 220°C. The reaction is preferably carried out under atmosphere of carbon dioxide, or under an inert gas, such as nitrogen.

Depending on the activity of the catalyst and the reaction conditions, the yield of dendritic polyglycerol In step (d) can be of at least 50 wt.%, preferably at least 60 wt.%; more preferably at least 80 wt.%. The catalyst is preferably separated from the mixture in step (e) by filtration.

The dendritic polyglycerol thereby produced is soluble in water, EtOH, DMF and MeOH with slight variations based on the functional groups on the surface, and can advantageously be used as nanotransporter in many applications, ranging from heterogeneous catalysis to medecine via encapsulation of highly hydrophobic and hydrophilic drugs or fluorescent compounds, from cosmetics via encapsulation of dyes, fragrances and vitamin E. Additional applications include, but are not limited to, its use as water softener in detergents or rinsing agents, or to bond antibodies, proteins or drugs containing primary amino groups.

### BRIEF DESCRIPTION OF THE FIGURES

For a fuller understanding of the nature of the present invention, reference is made to the following detailed description taken in conjunction with the accompanying drawings in which:
**Figure 1:** shows a reaction scheme of the formation of dendritic polyglycerol by catalytic polycondensation of glycerol according to the present invention.
**Figure 2****:** shows an NMR spectrum measured on a sample obtained from a process according to present invention.

### DETAILED DESCRIPTION OF THE INVENTION

As discussed in the BACKGROUND section, the glycerol polycondensation methods for the production of dendritic polyglycerol known to date generally have a rather low yield or have to be carried out in the vapour phase or dispersed in a solvent or aqueous medium. The present invention proposes a new process for producing hyperbranched polyglycerol from glycerol comprising the following steps:
(a) Adding in a vessel glycerol and a CaO-based catalyst,
(b) Flushing an inert gas, preferably carbon dioxide, to the resulting mixture and hermetically closing the reaction vessel, allowing pressure to build up from 1 to 10 bar above atmospheric, preferably from 2 to 6 bar;
(c) Heating the reaction mixture at a reaction temperature of at least 100°C and below its boiling point;
(d) Maintaining the reaction conditions until at least 40 wt.% of glycerol is polycondensed and converted into hyperbranched polyglycerol, with concomitant water formation, ,
(e) Separating the dendritic polyglycerol from other components present in the mixture; such as water, catalyst and reaction by-products such as hydroxyacids and glycerol carbonate esters obtained by condensation of deprotonated glycerol and carbon dioxide as well as minor amounts of cyclic compounds.

Figure 1 illustrates a reaction scheme associated with a process according to the present invention. The calcium based catalyst is a nanostructured Ca oxide known in the art obtained by treating a conventional calcium oxide to change and enhance the oxide catalytic activity. An example of nanostructured calcium oxide catalyst suitable for the present invention is described in Ruppert et al., Chem. Eur. J., 14, (2008) 2016-2024, and was obtained by thermal treatment of calcium hydroxide (Ca(OH)₂) under vacuum of 10⁻³ Torr and heated according to the following treatment: 25-350°C at a rate of 0.5°C min⁻¹ and maintaining the temperature of 350°C for 1 h; followed by further heating to 400°C with a rate of 1°C min⁻¹ and maintaining this temperature for 1 h. In another embodiment, the catalyst is obtained by calcination at atmospheric pressure of commercial Ca(OH)₂ in an oven at 350 to 450 °C for a period of up to 12 hours. Different temperatures and reaction times will yield CaO with different properties and degrees of activity.

A nanostructured Ca oxide catalyst as described in Ruppert *et al.* and useful for the present invention can alternatively be obtained by heating Ca(NO₃)₂ 4H₂O at a temperature of at least 500°C for at least 50 min. For example, catalysts with excellent activity can be obtained by heating Ca(NO₃)₂ 4H₂O for about 2 h at a heating rate of 10°C / min until a temperature of 650-700°C is reached an maintained. In yet an alternative preparation method, a solution in an organic solvent of Ca(OCH₃)₂ can be carried out by addition of deionized water at room temperature. In particular, if the organic solvent comprises methanol, hydrolysed calcium methoxide can thus be formed. The mixture can then be treated in an autoclave at elevated temperature and pressure. The temperature should be at least 200°C, preferably at least 220°C and the pressure is comprised between 10 and 60 bar, preferably between 25 and 50 bar. Calcium hydroxide is thus formed which can be converted into CaO by heating Ca(OH)₂ to a temperature of up to 450°C under reduced pressure of the order of 10⁻³-10⁻² Torr.

The thus obtained nanostructured CaO has a substantially higher activity than conventional CaO. In particular, it has a higher Lewis acidity than conventional CaO. As shown by A. Travert, A. Vimont, et al., Appl. Catal. A, 307 (2006) 98-107, which content is incorporated herein by reference, the blue shift, Δv8a, of the C=C stretching band of adsorbed pyridine as compared to the value of 1580 cm⁻¹ for gas-phase pyridine increases with increasing strength of the Lewis acid sites interacting with the pyridine molecule. When for conventional CaO, the blue shift, Δv8a, is generally comprised between 1 and 5 cm⁻¹, a nanostructured CaO catalyst according to the present invention is preferably characterized by a higher blue shift, Δv8a, value, generally comprised between 14 and 20 cm⁻¹, preferably between 16 and 19cm⁻¹, more preferably between 17.5 and 18.5 cm⁻¹. The specific surface area of the nanostructured CaO catalyst of the present invention is higher than conventional CaO, with a BET specific surface area for nanostructured CaO of at least 50m²/g, generally comprised between 60 and 100 m²/g, preferably between 65 and 90 m²/g, compared with the one of conventional CaO generally comprised between 5 and 10 m² /g as reported in R. H. Borgwardt, AlChE Journal, 31, (1985) 103-111. Nanostructured CaO catalyst is used in the present process in a powder form, with a mean particle size generally comprised between ranging in particle size from 1 to 90 µm made of agglomerate CaO nanoparticles.

The nanostructured catalyst is in the form of a powder of mean particle size generally smaller than 100 nm, preferably less than 50 nm, more preferably less than 20 nm. A mean particle size of about 10 nm was found to yield excellent results. In the dry state and at room temperature the particles may form micron-range agglomerates which break down when glycerol is heated. After the reaction is completed, and the temperature lowered, the nanoparticles coalesce to form larger aggregates, ready to catalyse the polycondensation of a next batch of glycerol. The mean particle size can conveniently be measured by a Laser light scattering according to ASTM D4464 - 10. Other techniques can of course be used.

The calcium based catalyst can be present in the mixture in any amount comprised between 4 and 30 mol.% with respect of the total weight of glycerol. It is preferably present between 5 and 25 mol.%, more preferably between 10 and 20 mol.%. The catalyst is mixed directly with glycerol and the mixture can be heated to the reaction temperature in a closed vessel after flushing with CO₂.

One great advantage of the present invention is that glycerol polycondensation occurs in the liquid phase with no need of any solvent or aqueous medium, which must subsequently be removed, and reaction occurs in one-pot with direct conversion of glycerol into hyperbranched polyglycerol. The reaction temperature in step (c) is preferably comprised between 180 and 280°C, more preferably between 200 and 250 °C, most preferably, between 215 and 225°C. The reaction is preferably carried out under a carbon dioxide atmosphere, preferably of 2 to 6 bar, or under an inert gas atmosphere such as nitrogen or argon. The reaction proceeds in a closed vessel to allow pressure to build up to 30 bar above atmospheric pressure due to release of water in gaseous phase. Hyperbranched polyglycerol yields of at least 50 wt.%, preferably at least 60 wt.%; more preferably at least 80 wt.% can be achieved with the present process, which is considerably higher than most prior art techniques comprising polycondensation of glycerol.

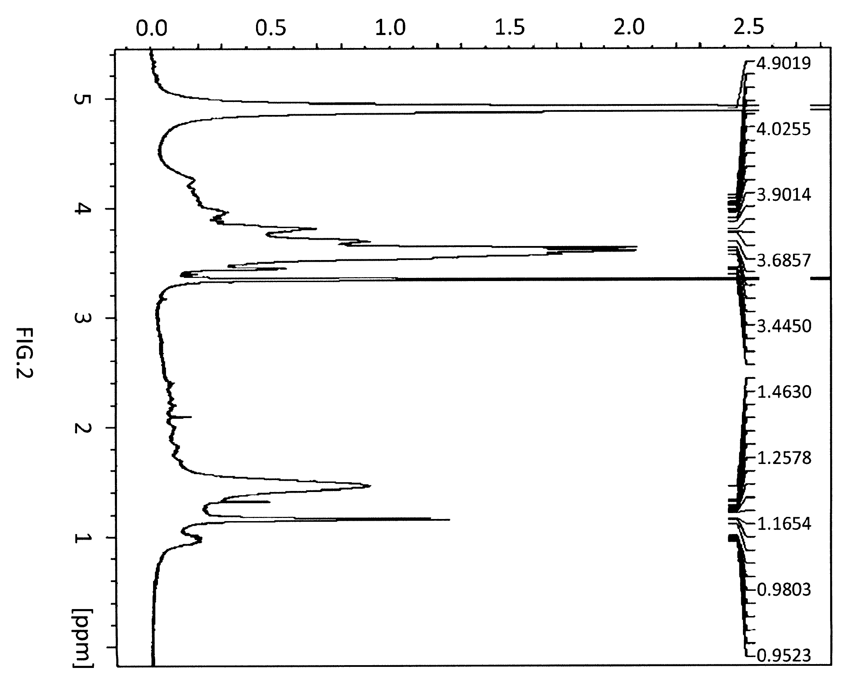

Figure 2 shows the proton NMR spectrum obtained on a sample produced according to the present invention. The sample obtained via glycerol polymerization at 220°C in the presence of 30 mol% nanostructured CaO for 5 h was simply dissolved in deuterated methanol and the NMR spectrum recorded according to known art in a Bruker 400 MHz spectrometer. The spectrum clearly indicates the presence of hyperbranched polyglycerol. A similar method was used and described in A. Sunder et al, "Hyperbranched polyether-polyols based on polyglycerol: polarity design by block copolymerization with propylene oxide", Macromolecules, 33 (2000) 309-314.

Beside formation of dendritic polyglycerol, NMR analysis revealed the formation of minor amounts of valuable hydroxyacids as well as of glycerol carbonate esters formed by fixation of CO₂ on glycerol. At the end of the reaction, the polymer is easily separated from the solid catalyst by simple centrifugation, and from the remaining by-products by distillation according to well known separation techniques used in the glycerol and polyglycerol industry. Dendritic polyglycerol thus produced can advantageously be used in any application including conversion into adhesisves and sealants, as support for controlled delivery and release of an active compound such as drugs and genes, derivatization with fatty acids for use in cosmetics, and the like, as described by Frey and co-workers reviewing the multiform applications of this versatile polyether dendritic polymer in "Hyperbranched Polyglycerols: From the Controlled Synthesis of Biocompatible Polyether Polyols to Multipurpose Applications" Acc. Chem. Res., 43 (2010), 129-141.

The use of nanostructured CaO as catalyst for the production of dendritic polyglycerol by polycondensation of glycerol is highly advantageous over prior art processes because:
- The reaction occurs in one-pot with direct conversion of glycerol into hyperbranched polyglycerol
- No solvent or aqueous medium is required, which must subsequently be removed
- No glycidol is employed in the reaction, with considerable economic, safety and health advantages
- Extremely high dendritic polyglycerol yields of the order of 95% and more can be achieved with the present process,
- Separation of dendritic polyglycerol from the solid catalyst and other by-products can be achieved simply by filtration or centrifugation to yield ready-to-use hyperbranched polyglycerol suitable for any further application.

## Claims

1. Process for producing dendritic polyglycerol from glycerol comprising the following steps:
(a) Adding in a vessel glycerol and a CaO-based catalyst,
(b) Flushing an inert gas, preferably carbon dioxide, to the resulting mixture and hermetically closing the reaction vessel, allowing pressure to build up from 1 to 10 bar above atmospheric, preferably from 2 to 6 bar;
(c) Heating the reaction mixture at a reaction temperature of at least 100°C and below its boiling point;
(d) Maintaining the reaction conditions until at least 40 wt.% of glycerol is polycondensed and converted into hyperbranched polyglycerol, with concomitant water formation,
(e) Separating the dendritic polyglycerol from other components present in the mixture; such as water, catalyst and reaction by-products such as hydroxyacids and glycerol carbonate esters and minor amounts of cyclic compounds.
**Characterized in that**, the calcium based catalyst is nanostructured calcium oxide in the form of a powder of mean particle size smaller than 100 nm measured according to ASTM D4464, and wherein the nanostructured Ca oxide has a blue shift, Δ*v*₈ₐ, of the C=C stretching band of adsorbed pyridine as compared to the value of 1580 cm⁻¹ for gas-phase pyridine comprised between 14 and 20 cm⁻¹

2. Process according to claim 1, wherein the nanostructured Ca oxide has a blue shift, Δ*v*₈ₐ, of the C=C stretching band of adsorbed pyridine as compared to the value of 1580 cm⁻¹ for gas-phase pyridine comprised between 16 and 19 cm⁻¹, preferably between 17.5 and 18.5 cm⁻¹.

3. Process according to claim 1 or 2, wherein the calcium based catalyst is present in the mixture in an amount comprised between 4 and 30 mol.% with respect of the total weight of glycerol and catalyst, preferably between 5 and 25 mol.%, more preferably between 10 and 20 mol.%.

4. Process according to any of the preceding claims, wherein the nanostructured Ca oxide catalyst has a BET specific surface greater than 50 m²/ g, preferably comprised between 60 and 100 m² / g.

5. Process according to any of the preceding claims, wherein the nanostructured Ca oxide is obtained by heating Ca(NO₃)₂ 4H₂O at a temperature of at least 500°C for at least 50 min or by heating Ca(OH)₂ at a temperature of at least 350°C for a period of up to 12 h.

6. Process according to any of the preceding claims, wherein the nanostructured Ca oxide is obtained by:
(a) Hydrolysing Ca(OCH₃)₂ in solution in an organic solvent by addition of water;
(b) Forming Ca(OH)₂ by treating the hydrolysed solution in an autoclave at a temperature of at least 200°C under elevated pressure;
(c) Converting the thus obtained Ca(OH)₂ into CaO by a heat treatment at at least 300°C under reduced pressure of not more than10⁻² Torr.

7. Process according to any of the preceding claims, wherein the calcium based catalyst is in a powder form of mean particle size smaller than 100 nm, preferably less than 50 nm, more preferably less than 20 nm.

8. Process according to any of the preceding claims, wherein the glycerol polycondensation is carried out in the melt phase, substantially free of solvents or of an additional aqueous medium other than the water formed during polycondensation.

9. Process according to any of the preceding claims, wherein the reaction temperature is at least 120°C, preferably at least 160°C, more preferably at least 220°C.

10. Process according to any of the preceding claims, wherein the reaction is carried out under nitrogen atmosphere or carbon dioxide, preferably under carbon dioxide.

11. Process according to any of the preceding claims, wherein the yield of dendritic polyglycerol is at least 50 wt.%, preferably at least 60 wt.%; more preferably at least 80 wt.%.

12. Process according to any of the preceding claims, wherein the catalyst is separated from the polyglycerol by filtration.

13. Process according to any of the preceding claims, wherein the dendritic polyglycerol thus obtained is used as support for controlled delivery and release of an active compound such as for drugs, catalysts, dyes, vitamines or genes, derivatized with fatty acids for use in cosmetics, as water softener in detergents or rinsing agents, or to bond antibodies, proteins or drugs containing primary amino groups, as well as adhesive or sealant or any other suitable application of this versatile polyether.

14. Use of a nanostructured calcium oxide catalyst as defined in any of claims 1 to 7 for the production of dendritic polyglycerol.

## Patentansprüche

1. Verfahren zur Herstellung von verzweigtem Polyglycerin aus Glycerin, die folgenden Schritte umfassend:
(a) Einbringen von Glycerin und eines Katalysators auf CaO-Basis in ein Gefäß,
(b) Spülen mit einem Inertgas, vorzugsweise mit Kohlenstoffdioxid, des daraus resultierenden Gemisches und hermetisches Verschließen des Reaktionsgefäßes, wodurch es möglich ist, einen Druck von 1 bis 10 bar über dem atmosphärischen Druck, vorzugsweise von 2 bis 6 bar aufzubauen;
(c) Erhitzen des Reaktionsgemisches auf eine Reaktionstemperatur von zumindest 100°C und unter dessen Siedepunkt;
(d) Beibehalten der Reaktionsbedingungen bis zumindest 40 Gew.-% vom Glycerin polykondensiert, und in hochverzweigtes Polyglycerin umgewandelt sind, mit der damit einhergehenden Bildung von Wasser,
(e) Trennen des verzweigten Polyglycerins von anderen Komponenten, die im Gemisch vorhanden sind; wie Wasser, Katalysator und Reaktionsnebenprodukte, wie Hydroxysäuren und Glycerincarbonat-Ester und geringerer Mengen an Ringverbindungen.
**Dadurch gekennzeichnet, dass** der auf Calcium basierende Katalysator ein nanostrukturiertes Calciumoxid in Form eines Pulvers mit einer Hauptpartikelgröße von kleiner als 100 nm ist, die gemäß ASTM-D-4464 gemessen wird, und wobei das nanostrukturierte Ca-Oxid eine Blauverschiebung, *Δv₈ₐ,* der C=C Streckschwingung von absorbiertem Pyridin aufweist, im Vergleich zu dem Wert von 1580 cm⁻¹ für Pyridin in der Gasphase zwischen 14 und 20 cm⁻¹

2. Verfahren nach Anspruch 1, wobei das nanostrukturierte Ca-Oxid eine Blauverschiebung, Δ*v₈ₐ,* der C=C Streckschwingung von absorbiertem Pyridin aufweist, im Vergleich zu dem Wert von 1580 cm⁻¹ für Pyridin in der Gasphase zwischen 14 und 20 cm⁻¹, vorzugsweise zwischen 17,5 und 18,5 cm⁻¹.

3. Verfahren nach Anspruch 1 oder 2, wobei der auf Calcium basierende Katalysator in einer Menge im Gemisch vorhanden ist, die zwischen 4 und 30 Mol-% im Vergleich zum Gesamtgewicht von Glycerin und Katalysator liegt, vorzugsweise zwischen 5 und 25 Mol-%, und am besten zwischen 10 und 20 Mol-%.

4. Verfahren nach irgendeinem der vorherigen Ansprüche, wobei der nanostrukturierte Ca-Oxid Katalysator eine BET-Oberfläche größer als 50 m²/g, und vorzugsweise zwischen 60 und 100 m²/g aufweist.

5. Verfahren nach irgendeinem der vorherigen Ansprüche, wobei man das nanostrukturierte Ca-Oxid durch Erhitzen von Ca(NO₃)₂4H₂O bei einer Temperatur von zumindest 500°C zumindest 50 Minuten lang, oder durch Erhitzen von Ca(OH)₂ bei einer Temperatur von zumindest 350°C für einen Zeitraum von bis zu 12 Stunden erhält.

6. Verfahren nach irgendeinem der vorherigen Ansprüche, wobei man das nanostrukturierte Ca-Oxid wie folgt erhält:
(a) Hydrolysieren von Ca(OCH₃)₂ in einer Lösung in einem organischen Lösungsmittel durch Beigeben von Wasser;
(b) Bilden von Ca(OH)₂ durch Behandeln der hydrolysierten Lösung in einem Autoklav bei einer Temperatur von zumindest 200°C unter erhöhtem Druck;
(c) Umwandeln des auf diese Art erhaltenen Ca(OH)₂ in CaO durch eine Wärmebehandlung bei zumindest 300°C unter verringertem Druck von nicht mehr als 10⁻² Torr.

7. Verfahren nach irgendeinem der vorherigen Ansprüche, wobei der auf Calcium basierende Katalysator eine Pulverform aufweist, mit einer Hauptpartikelgröße kleiner als 100 nm, vorzugsweise kleiner als 50 nm und am besten kleiner als 20 nm.

8. Verfahren nach irgendeinem der vorherigen Ansprüche, wobei die Glycerin-Polykondensation in der Schmelzphase durchgeführt wird, im Wesentlichen frei von Lösungsmitteln oder eines anderen zusätzlichen wässrigen Mediums, als Wasser, das während der Polykondensation gebildet wird.

9. Verfahren nach irgendeinem der vorherigen Ansprüche, wobei die Reaktionstemperatur zumindest 120°C, vorzugsweise zumindest 160°C und am besten zumindest 220°C beträgt.

10. Verfahren nach irgendeinem der vorherigen Ansprüche, wobei die Reaktion unter Stickstoffatmosphäre oder Kohlenstoffdioxid, und vorzugsweise unter Kohlenstoffdioxid durchgeführt wird.

11. Verfahren nach irgendeinem der vorherigen Ansprüche, wobei der Ertrag des verzweigten Polyglycerins zumindest 50 Gew.-%, vorzugsweise zumindest 60 Gew.-% und am besten zumindest 80 Gew.-% beträgt.

12. Verfahren nach irgendeinem der vorherigen Ansprüche, wobei der Katalysator durch Filterung vom Polyglycerin getrennt wird.

13. Verfahren nach irgendeinem der vorherigen Ansprüche, wobei das so erhaltene verzweigte Polyglycerin als Träger für die kontrollierte Bereitstellung und Freigabe einer aktiven Verbindung wie für Medikamente, Katalysatoren, Farbstoffe, Vitamine oder Gene verwendet werden, die mit fettigen Säuren für die Verwendung in der Kosmetik, als Wasserenthärter in Reinigungsmitteln oder Spülmittel abgeleitet werden, oder Verbund-Antikörper, Proteine oder Medikamente umfassende Aminogruppen, sowie Haftmittel oder Dichtmittel oder irgendeine andere Anwendung dieses vielfältigen Polyethers.

14. Verwendung eines nanostrukturierten Calciumoxid-Katalysators nach irgendeinem der Ansprüche 1 bis 7, zur Herstellung von verzweigtem Polyglycerin.

## Revendications

1. Procédé de production de polyglycérol dendritique à partir de glycérol comprenant les étapes suivantes :
(a) addition de glycérol et d'un catalyseur à base de CaO dans un récipient,
(b) balayage avec un gaz inerte, de préférence, du dioxyde de carbone, du mélange obtenu et fermeture hermétique du récipient réactionnel, permettant l'instauration d'une pression de 1 à 10 bars au-dessus de la pression atmosphérique, de préférence de 2 à 6 bars ;
(c) chauffage du mélange réactionnel à une température réactionnelle d'au moins 100°C et en-dessous de son point d'ébullition ;
(d) maintien des conditions réactionnelles jusqu'à ce qu'au moins 40% en poids de glycérol soient polycondensés et convertis en polyglycérol hyper-ramifié, avec formation concomitante d'eau,
(e) séparation du polyglycérol dendritique des autres composants présents dans le mélange tels que l'eau, un catalyseur et des sous-produits réactionnels tels que des hydroxy-acides et des esters de carbonate de glycérol et des quantités mineures de composés cycliques,
**caractérisé en ce que** le catalyseur à base de calcium est un oxyde de calcium nanostructuré sous la forme d'une poudre présentant une taille moyenne de particules inférieure à 100 nm mesurée selon la norme ASTM D4464, et dans lequel l'oxyde de Ca nanostructuré a un déplacement vers le bleu, Δv_{8a,} de la bande d'élongation C=C de la pyridine adsorbée par rapport à la valeur de 1580 cm⁻¹ pour la pyridine en phase gazeuse compris entre 14 et 20 cm⁻¹.

2. Procédé selon la revendication 1, dans lequel l'oxyde de Ca nanostructuré a un déplacement vers le bleu Δv₈ₐ, de la bande d'élongation C=C de la pyridine adsorbée par rapport à la valeur de 1580 cm⁻¹ pour la pyridine en phase gazeuse compris entre 16 et 19 cm⁻¹, de préférence entre 17,5 et 18,5 cm⁻¹.

3. Procédé selon la revendication 1 ou 2, dans lequel le catalyseur à base de calcium est présent dans le mélange en une quantité comprise entre 4 et 30% en mole par rapport au poids total de glycérol et de catalyseur, de préférence entre 5 et 25% en mole, de manière davantage préférée entre 10 et 20% en mole.

4. Procédé selon l'une quelconque des revendications précédentes, dans lequel le catalyseur oxyde de Ca nanostructuré a une surface spécifique BET supérieure à 50 m²/g, de préférence comprise entre 60 et 100 m²/g.

5. Procédé selon l'une quelconque des revendications précédentes, dans lequel l'oxyde de Ca nanostructuré est obtenu par chauffage du Ca(NO₃)₂ 4H₂O à une température d'au moins 500°C pendant au moins 50 min ou par chauffage du Ca(OH)₂ à une température d'au moins 350°C pendant une période allant jusqu'à 12 h.

6. Procédé selon l'une quelconque des revendications précédentes, dans lequel l'oxyde de Ça nanostructuré est obtenu par :
(a) hydrolyse du Ca (OCH₃)₂ en solution dans un solvant organique par addition d'eau ;
(b) formation de Ca(OH)₂ par traitement de la solution hydrolysée dans un autoclave à une température d'au moins 200°C à pression élevée ;
(c) conversion du Ca(OH)₂ ainsi obtenu en CaO par traitement thermique à au moins 300°C à une pression réduite ne dépassant pas 10⁻² torr.

7. Procédé selon l'une quelconque des revendications précédentes, dans lequel le catalyseur à base de calcium est sous une forme de poudre de particules ayant une taille moyenne inférieure à 100 nm, de préférence inférieure à 50 nm, de manière davantage préférée, inférieure à 20 nm.

8. Procédé selon l'une quelconque des revendications précédentes, dans lequel la polycondensation du glycérol est réalisée dans la phase de fusion, substantiellement sans solvants ou dans un milieu aqueux supplémentaire autre que l'eau formée pendant la polycondensation.

9. Procédé selon l'une quelconque des revendications précédentes, dans lequel la température réactionnelle est d'au moins 120°C, de préférence d'au moins 160°C, de manière davantage préférée, d'au moins 220°C.

10. Procédé selon l'une quelconque des revendications précédentes, dans lequel la réaction est réalisée sous atmosphère d'azote ou de dioxyde de carbone, de préférence sous dioxyde de carbone.

11. Procédé selon l'une quelconque des revendications précédentes, dans lequel le rendement du polyglycérol dendritique est d'au moins 50% en poids, de préférence d'au moins 60% en poids, de manière davantage préférée, d'au moins 80% en poids.

12. Procédé selon l'une quelconque des revendications précédentes, dans lequel le catalyseur est séparé du polyglycérol par filtration.

13. Procédé selon l'une quelconque des revendications précédentes, dans lequel le polyglycérol dendritique ainsi obtenu est utilisé comme support pour la distribution et la libération contrôlées d'un composé actif tel que des médicaments, des catalyseurs, des colorants, des vitamines ou des gènes, dérivatisés avec des acides gras pour leur utilisation dans des cosmétiques, comme adoucisseur d'eau dans les détergents ou agents de rinçage ou pour lier des anticorps, protéines ou médicaments contenant des groupes amino primaires ainsi que comme adhésif ou agent d'étanchéité ou toute autre application appropriée de ce polyéther polyvalent.

14. Utilisation d'un catalyseur d'oxyde de calcium nanostructuré tel que défini dans l'une quelconque des revendications 1 à 7, pour la production de polyglycérol dendritique.
